Europäisches Patentamt

European Patent Office

Office européen des brevets

(11) Publication number: **0 103 481**
A1

## (12) EUROPEAN PATENT APPLICATION

(21) Application number: **83305327.5**

(22) Date of filing: **13.09.83**

(51) Int. Cl.³: **A 61 B 17/00**

(30) Priority: 14.09.82 AU 5873/82
20.09.82 AU 5961/82
29.09.82 AU 6108/82
30.09.82 AU 6144/82
30.09.82 AU 6145/82
06.10.82 AU 6217/82

(43) Date of publication of application: **21.03.84**
Bulletin 84/12

(84) Designated Contracting States: **AT BE CH DE FR GB IT LI LU NL SE**

(71) Applicant: **AINSWORTH NOMINEES PROPRIETARY LIMITED, 130 Wattle Valley Road, Camberwell Victoria 3124 (AU)**

(72) Inventor: **Ainsworth, Geoffrey Hugh, 5 Wingan Avenue, Camberwell Victoria 3124 (AU)**

(74) Representative: **Hughes, Brian Patrick et al, Graham Watt & Co. Riverhead, Sevenoaks, Kent TN13 2BN (GB)**

(54) Treatment and reduction of obesity in humans.

(57) An anti-obesity device is self-contained and capable of being swallowed and received within the stomach. The device comprises an envelope (1) capable of dilation within the stomach by a variety of means such as the rupturing of a capsule (2) containing water which then reacts with a mixture (4) of sodium bicarbonate and citric acid to evolve carbon dioxide.

- 1 -

"TREATMENT AND REDUCTION OF OBESITY IN HUMANS"

This invention relates to the treatment and reduction of obesity in humans.

A conservative estimate has it that about one in five persons suffer from a weight problem that could, at least in part, be alleviated by dietary control. The major difficulty, however, is often the exercise of that control.

Many remedies have been and doubtless will continue to be suggested, which purport to assist in the exercise of that control. Of these, mention is made of dietary pills as a substitute for food. This has met with little success, principally because of the failure to recognise the psychological/physiological comfort to be had from consuming "real" food. Coupled with the consumption are the visual effects of the food, not to mention the satisfied feeling that can mark the end of a meal. Put another way, there is no "enjoyment" as such attendant upon the swallowing of a pill in lieu of a proper meal. In time, therefore, the dieter may well develop a real craving for a proper meal, and that could lead to the diet being broken and ultimately altogether abandoned.

Other techniques rely for their success on will-power, but suffice it to say not everyone is blessed with an abundance of this characteristic. Hypnosis, for example, has been used. The discipline necessary to ensure success with this method is very often considered to be, as it were, not worth the cure.

In surgical treatment of obesity, the abdominal cavity is exposed to allow reconstruction of the digestive tract, in essence, to reduce the internal surface area available for food digestion or absorption of digested substances. The former technique, a gastric bypass, may be accomplished by anastomosing a minor portion of the stomach to the jejunum while leaving the major portion connected to the duodenum. The latter of these techniques, an intestinal bypass, involves the short-circuiting of a majority of the combined lengths of the jejunum and ileum by connecting the first part of the jejunum to the last part of the ileum.

Both of these operations are deemed to involve such high risk to the patient that they are considered only as a lifesaving undertaking for morbidly obese individuals.

Beyond statistically significant operative and overall mortality rates, reported complications following the gastric bypass include marginal ulcers and wound infections. The intestinal bypass involves similar mortality rates and, reportedly, a greater number of postoperative complications and side effects. These include pulmonary emboli, wound infections, gastrointestinal hemorrhage, renal failure, and numerous other disorders. The nature, severity, and frequency of these problems have in fact led to doubts as to the advisability of the techniques for treatment of obesity.

Another and more recent surgical measure entails an operation in which the stomach is literally stapled to reduce its capacity to take in food. But, apart from the trauma of the operation, there have again been instances of very bad side effects, indeed so much so that the operation has in effect been "undone".

Another approach to correcting obesity involves reducing the desire for food. This can be accomplished by partially filling the stomach so as to produce the sensation of being "filled-up". One way of accomplishing this is to place an inflatable, elastomeric bag in the stomach and inflate the bag with fluid.

Inflatable bag and tube combination devices are known in the field of medicine and have experienced use by the medical profession in the treatment of gastric disorders such as stomach ulcers and hemorrhages in the upper gastrointestinal tract. Cook, U.S. Pat. No. 3,227,154 discloses a device in which the bag must be fully inflated so that its impressionable-settable outer surface can make impressions in the walls of cavities which have restricted outlets. Another use of inflatable bag and tube combination devices as disclosed by Gawura, U.S. Pat. No. 3,768,484 and Seaman, U.S. Pat. No. 3,174,481, is to transfer and retain a cooling solution at a particular internal location. The bag acts merely as a container for this solution and the characteristic of being inflatable allows a single bag to be used with a wide range of cavity sizes. These prior art references all disclose tubes with a plurality of passageways and a liquid to inflate the bag.

In all of these situations, the use of the inflatable bag is of a short duration; once the diagnosis or treatment has been performed, the bag is removed from the human being's body. These types of applications do not require, nor do they benefit, from the bag remaining within the human being.

Berman et al, U.S. Pat. No. 4,133,315 discloses a method of reducing obesity in a person by decreasing the desire for food which comprises the following steps. First, an inflatable bag is placed in a person's stomach. Next, the bag is caused to be inflated to a sufficient size to cause a suppression of the person's appetite. Finally, the bag is allowed to remain in the stomach while the person is eating. The bag is sized for positioning in the human being's stomach and has a single opening to which a tube is attached. Once the human being has eaten, the bag can be deflated all at once, or it can be deflated gradually over a period of a few hours so as to simulate the condition of digestion occurring and the gradual reduction of stomach contents. This arrangement, by virtue of the bag/tube attachment, means that the bag is not freely positioned in the stomach. It is thus not as versatile and useful as it otherwise might be.

Also, although swallowing is described as one means of placement of the bag in the stomach, this step is difficult and it may instead prove necessary to introduce the bag under medical supervision and only then after the patient has been sedated. In this inserting, care must be taken if abrasions and retching are to be avoided.. Moreover, this method necessarily entails the protrusion of the tube from the patient's mouth, an inconvenience at best and perhaps also a source of embarrassment.

Harboe et al, GB Pat. Appln. No. 2 090 747 discloses an apparatus for introducing an expandable or inflatable foreign member through the mouth and the gullet for freely detached positioning in the human stomach. See also "The Lancet", 23 January, 1982, pp. 148-199. This apparatus comprises a combination of an elongated introducing member which can be positioned stretching through the mouth and the gullet having a first end projecting from the mouth and a second end projecting into the stomach, and an acid-resistant hollow foreign member which can be filled or inflated to a gas-, air- and/or liquid-filled yielding member having a uniform wall thickness. The introducing member comprises an elastic introducing tube which at its first end is provided with a means for supplying pressurized gas air and/or liquid for expanding or inflating the hollow member, and which at its second end is provided with a withdrawable packing member being positioned in a one way valve member which constitutes an integral part of the hollow member. The introducing tube is encased within an outer elastic tube which connects the lower end of the means for supplying gas, air and/or liquid with the upper end of the valve member, the outer tube acting as an abutment on the edge of the valve member. The packing member is preferably a thickening of or a thickened portion of the elastic introducing tube itself.

In operation, the outer and inner tubes together with the attached foreign member are introduced through the patient's mouth and gullet and advanced until the foreign member portion reaches the stomach.

The foreign member is then expanded or inflated by means of the injection device, thus filling the foreign member with air, gas and/or liquid. The foreign member is then ready to be left in the stomach, and the introducing portion is detached from the foreign member portion by pulling back in an upwards or axial direction. Difficulties are believed to have been encountered in detaching the introducing portion from the foreign member portion, principally due to a difficulty in locating satsifactory plastics materials for the task at hand. On the one hand the material must be such as to facilitate ready detachment of the introducing portion, but on the other hand, it must be sufficiently strong so that there is no inadvertent separation or escape of air, gas and/or liquid during inflation, when the introducing tube is subject to a necessarily high fluid pressure because of its small cross-sectional configuration. It is also believed that some discomfort might occur when the introducing portion is pulled back, for then the foreign member portions will tend to jam against the upper opening to the stomach. Moreover, this technique suffers from the psychological disadvantage, not to mention discomfort, attendant upon there being, for some time, a foreign body, namely the introducing portion, extending out through the mouth of the user. Still further, the apparatus due to the number of parts required and the need for close working tolerances between these inter-relating parts does not readily lend itself to ease or economy of manufacture.

With the foregoing in view, an object of the invention is to provide for a similar treatment to that described above, but in a way that is generally simpler and more convenient, and which tends to reduce the subject's discomfort.

The present invention is a physiologically acceptable self-contained device capable of being completely swallowed by and of being received within the stomach of a human being, said device including a ductless envelope adapted to dilate within the stomach.

For the purposes of this specification the masculine includes the feminine and vice versa.

In one form of the invention there is provided a physiologically acceptable self-contained device capable of being completely swallowed by and of being received within the stomach of a human being, said device including an envelope adapted and after reception in the stomach to self-inflate and eventually to deflate, without requiring tubes, catheters or other external ducts, and after deflation to be excluded from the body by passage through the normal excretory system.

The dilation, expansion or self-inflation may be achieved by means of gas generated or released from within the envelope. For example, the envelope may contain a quantity of inert liquid such as trichlorofluoromethane, commonly known as "Freon 11" which, before the envelope is swallowed may be substantially in the liquid phase. However after the envelope is received within the stomach the heat of the body vapourizes the freon and the envelope dilates under the internal pressure of the freon gas.

Advantageously the envelope is made of a "TEFLON" plastics material.

The invention also provided a method of reducing obesity in a human, characterized by the step of completely swallowing a self-contained physiologically acceptable envelope which is received by the stomach to occupy therein a volume that would otherwise contain or be available to contain food, and to dilate so as to reduce the remaining available volume of the stomach.

The envelope may be swallowed while in a generally non-inflated condition. The term "generally non-inflated" as used herein includes levels of inflation, usually more or less insignificant, up to those at which a difficulty is encountered in the introduction - for example, as by swallowing - of the envelope into the stomach.

The term "allowing" is used herein to connote situations where inflation has already begun at the introduction stage, and this is allowed to continue in the stomach; and where, at or during the introduction stage, no inflation has taken place, and inflation is caused to be initiated when the envelope is in the stomach.

In a further embodiment, two compounds, capable of reacting one with the other to produce an inflating gas, are stored in the envelope in such manner that contact, and hence reaction, is prevented until such time as said contact is desired. The two compounds may be maintained apart prior to the reaction by a physical barrier, set up with the aid of means within or external to the envelope.

According to a still further embodiment, the envelope containing a first compound, is introduced into the stomach, whereafter fluid in the stomach is able to enter the envelope via a closable opening therein and react with the first compound to thereby produce an inflating gas, the opening in the envelope thereafter closing to enable inflation of the envelope to be effected.

Apparatus suitable for use in connection with the invention may comprise an inflatable envelope which houses a first compound and a second compound, said first and second compounds being capable of reacting one with the other to produce an inflating gas; and means within the envelope to prevent contact of said first and second compounds until such contact is desired; said apparatus for reducing obesity in a person, which apparatus comprises an inflatable envelope which houses a first compound and a second compound, said first and second compounds being capable of reacting one with the other to produce an inflating gas; and means within the envelope to prevent contact of said first and second compounds until such contact is desired; said envelope having no communicating tube or tubes by which a source of gas or liquid external to the body can be introduced into the envelope to thereby inflate the envelope; such envelope being so adapted as to be readily receivable in the stomach of the person in a non-inflated condition; and said first and second components and hence said inflating gas each being present in such an amount as to inflate said envelope on the reaction of said first and second compounds. The envelope will usually be a balloon or bag made of say "Teflon" or "Neoprene".

Bearing in mind the envelope is to be swallowed, any significant amount of inflation in advance of swallowing is to be avoided or minimised if possible, for

0103481

- 12 -

otherwise a difficulty in the 'administration' of the envelope is likely to be encountered.  The hitherto recited 'means within the envelope' may serve this end.

This means a) may take the form of a coating (for instance, sugar) on one and/or other of the first and second compounds, the coating being designed to delay the onset of the reaction for a predetermined period of time;  or b) may be a rupturable or soluble capsule, which capsule could, if desired, be located in a set position within the envelope - for example, the neck;  or, c) may be an inner wall serving as a physical separating means for the first and second compounds and being readily rupturable on demand, or d) may combine two or more of the foregoing.  Clearly, the means can however be otherwise provided, mindful of the end objective.

The apparatus aspect of the invention makes provision for an inflatable envelope which houses a first compound and a second compound, said first and second compounds being capable of reacting one with the other to produce an inflating gas;  and means outside of the envelope, but associated therewith, to prevent contact of said first and second compounds until such contact is desired;  said envelope having no communicating tube or tubes by which a source of gas or liquid external to the body can be introduced into the envelope to thereby

- 13 -

inflate the envelope; said envelope being so adapted as to be readily receivable in the stomach of the person in a non-inflated condition; and said first and second compounds and hence said inflating gas each being present in such an amount as to inflate said envelope on the reaction of said first and second compounds. The outside means preferably serves to divide the envelope, with said first compound being located in one part and said second compound in (the) or (an)other part.

This means may take the form of a clip or tie which is able to pinch the envelope and thereby create at least two parts therewithin. The clip or tie can be removed just prior to swallowing of the envelope; or, it can be made of a soluble or thawable material, in which case it too is swallowed, whereafter the contents react upon dissolution or thawing of the clip or tie and subsequent contact of the contents. By another arrangement, the envelope includes a temporary fold line about which it can be folded and spot glued, for instance. The glue thus constitutes this means. It may be, in effect, torn off as desired, or as with the clip or tie be soluble in the stomach. If soluble, the glue will be non-toxic at least in the amount used on the envelope.

In the foregoing aspects, the envelope can moreover contain a liquid - one of the two reactants, or an additional non-reactive component - as well as the gas. This arrangement may make for an easier excretion of the bag after its deflation, because a more complete deflation is considered possible - more complete, that is, than when the bag houses gas only.

The expression "compound" as used herein is to be understood, in each and all cases, as encompassing single compounds and mixtures of compounds, that is, compositions. The compound(s) may be (a) solid(s), liquid(s) or gas(es).

Examples of the first and second compounds are water and a mixture of sodium bicarbonate or calcium carbonate and citric acid. Reaction of the two compounds gives rise to the evolution of carbon dioxide. Many other combinations may likewise be put to use to the same end.

Other non-toxic gases may, of course, also be produced, by the judicious selection of the first and second compounds, and suffice it to say the respective amounts of the two compounds are such that the gas ultimately produced is sufficient to inflate the balloon or like envelope.

Any solid reactant may be delay coated, so that reaction to produce the inflating gas is further controlled. The envelope may be of any material tailored for the task of expanding or unfolding under the pressure created by the inflating gas. It is also non-toxic, as with the gas; and resistant, moreover, to the conditions prevailing in the stomach, for a predetermined period of time.

The inflating gas, at least in the amounts employed to inflate the envelope, will be generally of a non-toxic nature. The term "non-toxic" as used herein, be it in relation to the gas or otherwise, is to be understood in this sense.

The envelope may contain a compound which is capable of assuming a gaseous state when the envelope is in the stomach; said envelope having no communicating tube or tubes by which a source of gas or liquid external to the body can be introduced into the envelope to thereby inflate the envelope; said envelope being so adaptive as to be readily receivable in the stomach of the person in a non-inflated condition, and said compound being present in such an amount as to inflate said envelope when said envelope is in the stomach and said compound has assumed said gaseous state.

By way of example only, mention is made of trichlorofluoromethane, otherwise commonly known as Freon 11. This particular compound has a boiling point of about $23.7^{\circ}C$, which means it needs to be stored in the member under normal chill or freeze conditions and yet will readily vapourize when under the influence of body temperature. Freon 11, in the amounts contemplated in this context, is non-toxic.

The envelope can be pre-sealed; or, it can be sealed by the user, for instance by a simple tying operation as and when it is to be 'taken'; or, it can be self-sealing in the stomach, as for example with the aid of a suitable valve in the envelope, which closes on the attainment of a certain inflating pressure in the envelope.

A number of smaller inflated envelopes may in some cases be used instead of one larger envelope occupying the same space in the stomach. Although these smaller envelopes are not likely to deflate at exactly one and the same time despite an inbuilt, predetermined lifetime of the neoprene or like material of which the envelopes are made, it is nonetheless preferable to employ envelopes that deflate at progressive intervals. Three envelopes, for instance, could be used - each, say, colour coded - and as one deflates and is subsequently excreted, a further like coloured envelope could be swallowed; or, if the treatment is having the desired effect - to wit, a reduction in weight - the excreted envelope need not be replaced. On the other hand, if only one envelope is to be used, this naturally may vary in size from person to person depending on the desired severity of the treatment or the particular stage of treatment.

Swallowing of·the envelope(s) can be undertaken with or without medical supervision, and if thus supervised the throat may be anaesthetised if deemed desirable by the medical practitioner.

A pack is also within the ambit of the present invention, said pack containing (I) in one form -

i)      the envelope, e.g. a balloon;

ii)     the aforementioned first compound;

iii)    the aforementioned second compound;

iv)     the aforementioned 'external means';
        and

v)      instructions for the placement of ii)
        and iii) in the bag and for the use of
        iv) to prevent contact of ii) and iii)
        until desired;

or (II) in another form -

vi)     the aforementioned i), ii), iii) and v);

vii)    optionally the aforementioned 'internal
        means';  and

viii)   where vii) is not present, v) includes
        instructions also for the creation of vii);

or (iii) in another form -

ix)    the aforementioned i);

x)    the aforementioned gaseous assuming compound;  and

xi)    instructions for the introduction of x) into ix) in a non-gaseous state and for the use of ix) and x).

Embodiments of the present invention will now be described, by way of example, with reference to the accompanying drawings, in which:-

Figs. 1, 2 and 3, 4, 5, 6, 8, 9, 10, 12, 13, 14, and 15 illustrate respective embodiments of the present invention;

Figs. 7 and 11 illustrate packs incorporating embodiments of the present invention;  and

Fig. 16 illustrates the invention in use.

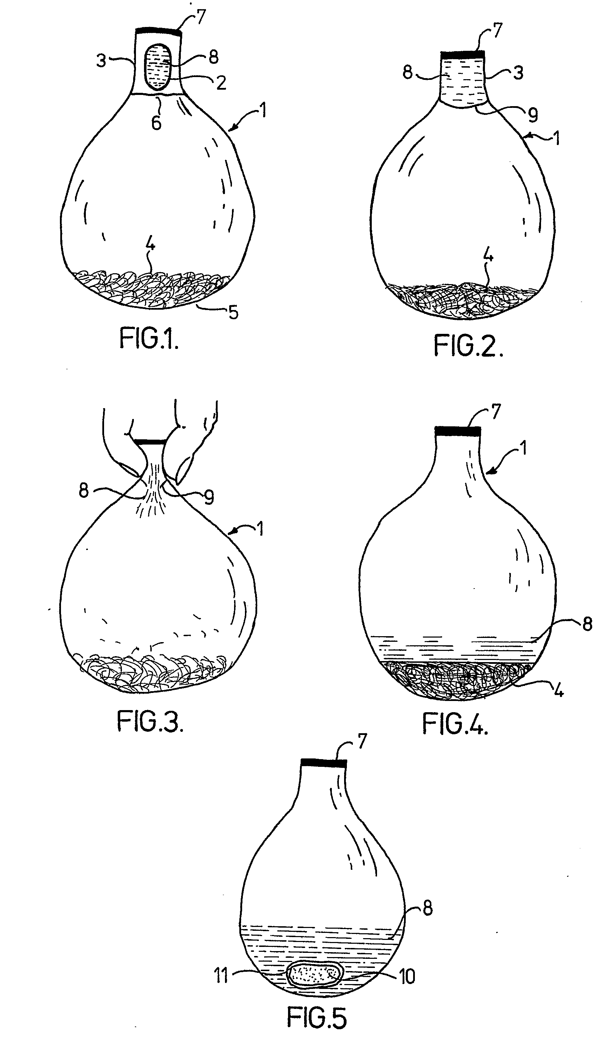

In Fig. 1, the envelope, a balloon - generally designated as 1 - houses a capsule 2 in the neck 3 and a solid 4 in the bottom 5.  The capsule 2 is held captive by any suitable means, and includes a weakened lower zone 6 designed to rupture on a firm squeezing thereof.  The balloon 1 is sealed at the top 7.  At this stage, it is not inflated.

The capsule 2 houses water 8 and the solid 4 is a mixture of sodium bicarbonate and citric acid. On rupturing the capsule 2, at 6, the water 8 mixes with the solid 4 and carbon dioxide is evolved. This gas inflates the balloon 1. Under these circumstances, it is imperative that the balloon 1 be swallowed quickly following capsule rupture; otherwise, it is conceivable that the bag could stick in the throat, or even be impossible to swallow.

In Figs. 2 and 3 the balloon 1 incorporates a frangible wall 9, so that the water 8 is bounded by the neck 3, the seal 7 and the wall 9 and not the capsule 2.

A yet further embodiment - not illustrated, however - could incorporate both the capsule 2 and the wall 9.

In Fig. 4, the water 8 and the solid 4 are in 'apparent' contact with each other. But, the solid particles are sugar or like coated to a thickness designed to delay the onset of the reaction for a predetermined period of time.

This particular arrangement does not lend itself to storage of the sealed balloon for any significant length of time, and it is envisaged that the balloon be sealed by the user and swallowed shortly thereafter.

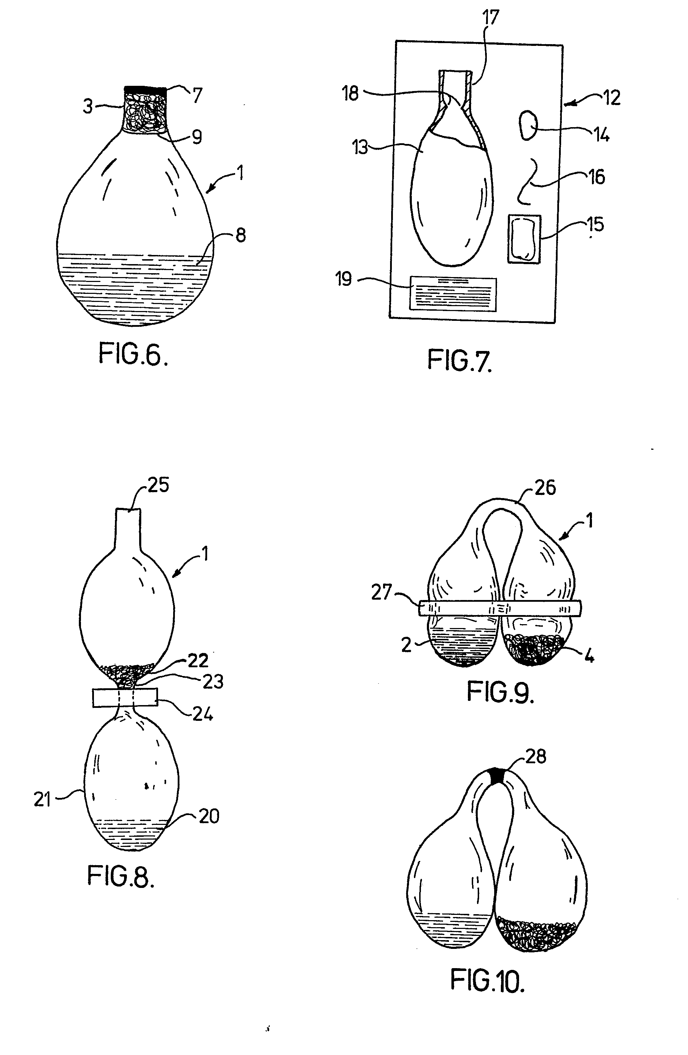

In Fig. 5, the solid assumes the form of a pill having a core 10 of the active ingredient(s) and a delay coating 11, again of sugar or the like.

Fig. 6 shows a more or less reverse arrangement to that of Fig. 2, with the solid 4 at the top and the liquid at the bottom. In this case, however, an excess of liquid is employed so that the liquid can ultimately assist during deflation.

In Fig. 7, the pack - generally designed as 12 - includes a balloon 13, a capsule 14, a sachet 15 and a tie 16. The balloon neck 12 is stepped at 18 to arrest downwards movement of the capsule 14. Instructions 19 are also included.

To use the pack, the sachet contents - say, the bicarbonate/citric acid mix - are emptied into the balloon, the capsule inserted and the neck tied. The pack can be stored as such until ready for final use, or, more likely, the capsule ruptured and the balloon (and all its contents) swallowed.

In Fig. 8, the balloon houses water 20 in the bottom 21 and a particulate solid 22 - for example, of sodium bicarbonate and citric acid - above the waist 23. The waist 23 is closed off by a clip 24, to prevent the solid 22 dropping through the waist and making contact with the water 20. The balloon is sealed as at 25 but, at this stage, is not inflated.

The clip 24 is made to dissolve when in the

- 21 -

stomach, whereupon the solid 22 and water 20 are free to mix and thus react to form $CO_2$. The carbon dioxide inflates the balloon 1.

In Fig. 9, the balloon 1 is folded at 26 and the clip 27 applied across the two halves of the balloon 1.

In Fig. 10, the clip is replaced by a spot of glue 28 which provides a temporary arrest to the unfolding of the balloon 1.

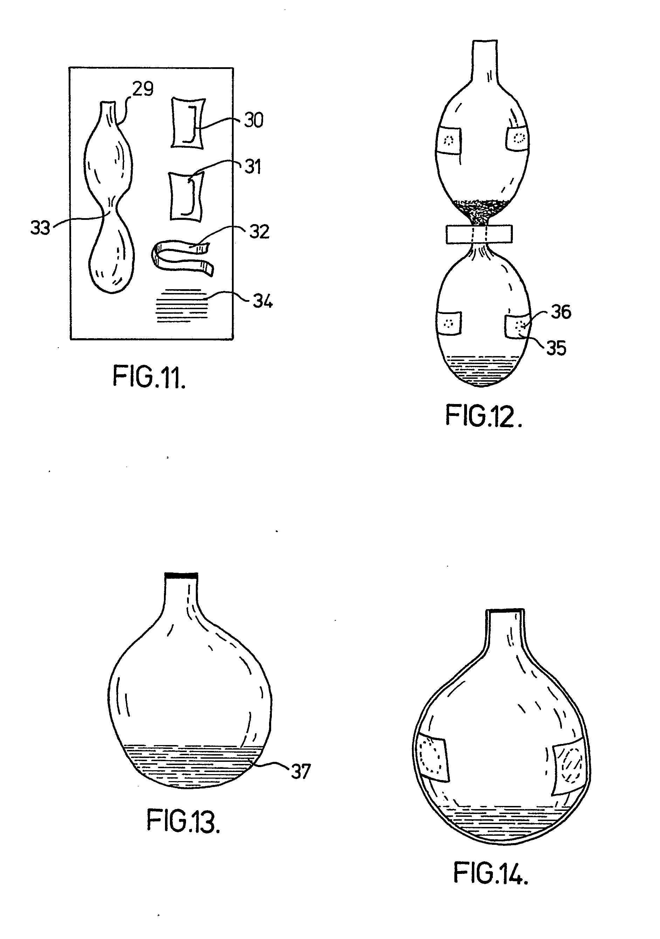

In Fig. 11, the pack includes a balloon 29, two sachets 30 and 31 and a soluble clip 32. The balloon is waisted at 33 to facilitate closure of that portion of the balloon. Instructions 34 are also included.

To use the pack, the sachet 30 contents - say, the water - are emptied into the balloon, the clip 32 applied across the waist 33, the sachet 31 contents added and the balloon sealed. The pack can be stored as such until ready for final use or, more likely, swallowed.

In Fig. 12, the balloon embodies a number of what are in effect patches 35 covering preformed holes 36. These patches are made of a less resistant material than that employed for the remainder of the bag, and this ensures that 'large' holes ultimately appear in the bag which in turn assist in the deflation of the bag. Contrastingly, with bag pin-holes as may occur when no patches are used, full or substantial deflation may be difficult.

In Fig. 13, the balloon contains a low boiling point liquid 37 such as Freon 11, characterized on the one hand by being readily storable at low (but not necessarily freeze) temperatures and, on the other, by boiling at normal body temperature. With this arrangement, some care may need to be taken in the handling of the balloon at the intake stage.

Fig. 14 introduces the additional patches/holes aspect of Fig. 12.

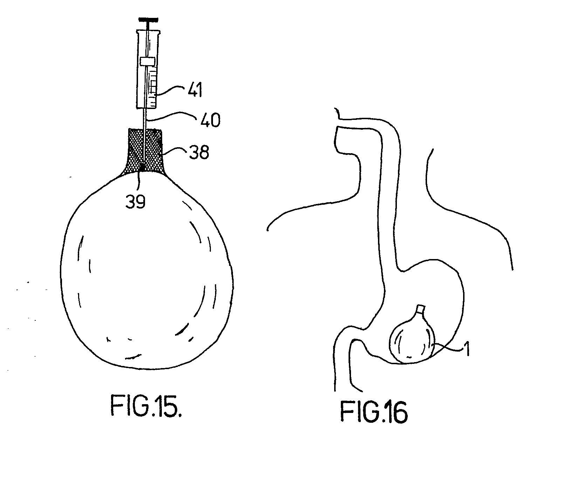

Fig. 15 depicts a balloon having an inbuilt valve 38. The valve 38 is of the type encountered in such items as footballs, baseballs etc. It is made of a suitable resiliently deformable rubber and has an axial, but normally closed, passage 39, which can be opened by for example the needle 40 of a syringe 41. In this particular case, the syringe 41 contains liquid Freon 11 for introduction under appropriate temperature conditions into the interior of the balloon. On withdrawal of the syringe 41, the passage 39 once again closes and the valve 38 hence presents a seal for the balloon.

It will be seen from the foregoing that the invention provides an eminently safe and comfortable means for reducing obesity in a human being, by reducing his effective capacity, and hence his desire, to eat and/or drink.

It is to be appreciated that various additions and/or modifications may be made to the foregoing, without in any way departing from the ambit of the invention.

0103481

- 24 -

CLAIMS:

1.    A physiologically acceptable self-contained device capable of being completely swallowed by and of being received within the stomach of a human being, said device including a ductless envelope adapted to dilate within the stomach.

2.    An envelope adapted to occupy part of the volume of a human stomach and to dilate itself therein so as to reduce the volume available for reception of food and/or drink.

3.    A physiologically acceptable self-contained device capable of being completely swallowed by and of being received non-permanently within the stomach of a human being, said device including an envelope adapted after reception or while in the stomach to dilate without requiring tubes or other external means of inflation.

4.    A physiologically acceptable self-contained device capable of being completely swallowed by and of being received within the stomach of a human being, said device including an envelope adapted and after reception in the stomach to self-inflate and eventually to deflate, without requiring tubes, catheters or other external ducts, and after deflation to be excluded from the body by passage through the normal excretory system.

5. A device as claimed in any one of the preceding claims, wherein the envelope contains an inert liquid material adapted to expand in response to body heat.

6. A device as claimed in claim 5, wherein the envelope is of a "TEFLON" plastics material and the liquid is trichlorofluoromethane.

7. A device as claimed in any one of claims 1 to 4, wherein the envelope contains two interactable substances and means permitting interaction of said substances when and only when the device is in the stomach.

8. A device as claimed in any one of claims 1 to 4, wherein the envelope contains a material reactable with stomach fluid, said envelope being adapted to admit such fluid to react with the material to produce inflating gas.

9. A physiologically acceptable self-contained device substantially as herein described with reference to any one of Figs. 1 to 16 of the accompanying drawings.

0103481

FIG.1.

FIG.2.

FIG.3.

FIG.4.

FIG.5

0103481

FIG.6.

FIG.7.

FIG.8.

FIG.9.

FIG.10.

0103481

FIG.11.

FIG.12.

FIG.13.

FIG.14.

0103481

FIG.15.

FIG.16

0103481

**European Patent Office**

Application number

EP 83 30 5327

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-4 246 893  (BERSON) | | A 61 B  17/00 |
| A,D | GB-A-2 090 747  (HARBOE) | | |

The present search report has been drawn up for all claims

| Place of search | Date of completion of the search | Examiner |
|---|---|---|
| THE HAGUE | 06-12-1983 | LOWE D. |

**TECHNICAL FIELDS SEARCHED (Int. Cl. ³)**

A 61 F
A 61 B
A 61 M
A 61 J

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82